(19) Europäisches Patentamt
European Patent Office
Office européen des brevets

(11) Publication number: 0 242 147 B1

(12) # EUROPEAN PATENT SPECIFICATION

(45) Date of publication of patent specification: **02.09.92**

(51) Int. Cl.5: **B01D 71/50**, B01D 53/22, C08G 63/68, C08G 64/00

(21) Application number: **87303173.6**

(22) Date of filing: **10.04.87**

(54) **The use of polycarbonate containing semipermeable membranes in the separation of gases.**

(30) Priority: **14.04.86 US 851758**

(43) Date of publication of application:
**21.10.87 Bulletin 87/43**

(45) Publication of the grant of the patent:
**02.09.92 Bulletin 92/36**

(84) Designated Contracting States:
**AT BE CH DE FR GB IT LI NL**

(56) References cited:
**GB-A- 1 059 945**
**US-A- 37 730**
**US-A- 3 028 365**
**US-A- 3 822 202**
**US-A- 4 022 944**

**PATENT ABSTRACTS OF JAPAN, vol. 7, no. 77 (C-159)[1222], 30th March 1983; & JP-A-58 8511 (TOYO BOSEKI K.K.) 18-01-1983**

**IDEM.**

(73) Proprietor: **THE DOW CHEMICAL COMPANY**
**2030 Dow Center Abbott Road P.O. Box 1967**
**Midland Michigan 48640-1967(US)**

(72) Inventor: **Anada, Joginder N.**
**30 Mountaire Place**
**Clayton California 94517(US)**
Inventor: **Feay, Darrell C.**
**67 La Cuesta**
**Orinda California 94563(US)**
Inventor: **Bales, Stephen E.**
**4409 Andre Street**
**Midland Michigan 48640(US)**
Inventor: **Jeanes, Thomas O.**
**2312 Robles Drive**
**Antioch California 94509(US)**

(74) Representative: **Burford, Anthony Frederick et al**
**W.H. Beck, Greener & Co. 7 Stone Buildings**
**Lincoln's Inn**
**London WC2A 3SZ(GB)**

EP 0 242 147 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**JOURNAL OF MEMBRANE SCIENCE, vol. 34, no. 2, 15th November 1987, (A.C.S. SOUTH-WEST REGIONAL MEETING, November 1986, Houston, Texas), pages 185-198, Elsevier Science Publishers B.V., Amsterdam, NL; N.MURUGANANDAM et al.: "Evaluation of substituted polycarbonates and a blend with polystyrene as gas separation membranes"**

## Description

This invention relates to semi-permeable membranes derived from polycarbonates wherein the polycarbonates are derived in a significant portion from tetrahalobisphenols. The invention further relates to the use of these membranes to separate certain gases, for example, oxygen from nitrogen, and carbon dioxide from methane.

In various industries, it is necessary or highly desirable to separate one component from another in a gaseous stream. Processes used to perform such separations include pressure swing absorption and membrane separations. In a membrane separation, a gaseous stream containing the components to be separated is contacted with a membrane, wherein the membrane separates two regions in a manner such that only those materials which permeate through the membrane can communicate from one region to the other. Such membranes are semi-permeable, in that one component of the gaseous mixture selectively permeates through the membrane at a rate much higher than one or more of the other components in the gaseous stream. The gaseous mixture is contacted with the membrane in a manner such that the selectively permeable species is preferentially transported through the membrane to the other region. It is to be noted that the component from which the selectively permeable species is to be separated may in fact permeate through the membrane at a much slower rate than the selectively permeable species. It is this difference in rates of permeation which is used to separate the gaseous species or reduce the concentration of the less selectively permeated species in the region to which the permeating gases permeate.

In such separations, the relative rate of permeation, that is, the difference in rate of permeation between the selectively permeating gas and the non-selectively permeating gas, is a major factor in the separation achieved. The higher the ratio of permeation of the selectively permeable gas over the non-selectively permeable gas, the better the membrane will perform. Therefore, it is desirable to have as high a ratio as possible.

Presently, membranes derived from acetate esters, for example cellulose triacetate, and olefins, for example polyethylene, polypropylene, poly-4-methylpentene-1, are used for gas separations. Among such separations are the separation of oxygen from nitrogen, and carbon dioxide from methane.

Some of the materials used in membranes suffer from certain disadvantages. One such problem is the inability to perform under extreme conditions, such as high temperatures and pressures. As a result, certain separations are limited by the membrane as to the temperatures and pressures which may be used.

What are needed are membranes with a high relative rate of permeation through the membrane of the selectively permeating species over the non-selectively permeating species. Further what is needed is a membrane which has enhanced mechanical strength so as to withstand more extreme temperatures and pressures.

EP-A-0037730 discloses filtration membranes for use in separating biological materials such as proteins and viruses. Reference is made to [4,4' - isopropylidene - bis (3,5 - dichlorophenylene) carbonate] but there is no specific example of a membrane comprising this polymer, and no reference is made to methods of gas separation.

JP-A-588511 discloses polycarbonate gas separation membranes. There is a general reference to halogenation of the membrane polymer, but no specific examples are given. The membrane disclosed shows a separation factor for $O_2/N_2$ of only 4.

US-A-4022944 discloses a process for making a thin membrane based on a bisphenol polycarbonate. There is no reference to halo-substitution of the bisphenol moieties and the membrane is reported to have a separation factor for $O_2/N_2$ of only about 5.

US-A-3028365 describes high molecular weight thermoplastic polycarbonates and methods for the manufacture thereof. There is a general reference to reaction of tetrachloro- or tetrabromo- substituted dihydroxydiphenyl propane with phosgene. However, there is no reference whatsoever to methods of gas separation.

GB-A-1059945 discloses polycarbonate gas separation membranes based on bisphenol A. There is a general reference to chloro- or bromo- substitution of the bisphenol moiety, but no specific examples are given. The membranes disclosed appear to show a separation factor for $O_2/N_2$ of only about 4.6.

The invention provides a method of separating a gas from a gas mixture, comprising

(a) contacting the gas mixture with one side of a gas separation membrane while maintaining a lower pressure on the other side of the membrane, said membrane comprising a thin discriminating layer of a polycarbonate polymer derived from a bisphenol corresponding to formula I

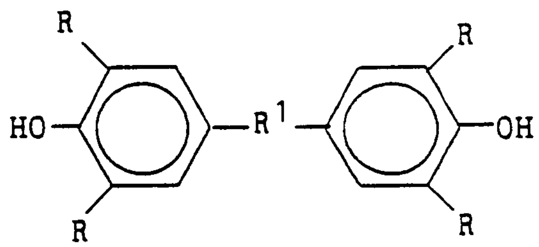

(I)

wherein R at each occurrence is independently H, Cl, Br, or $C_1$-$C_4$ alkyl and $R^1$ is -CO-, -S-, -$SO_2$-, -O-, a $C_1$-$C_6$ divalent hydrocarbon radical, a $C_1$-$C_6$ divalent fluorocarbon radical, or inertly substituted $C_1$-$C_6$ divalent hydrocarbon radical, with the proviso that at least 25 weight percent of the moieties derived from the bisphenol of formula I present in the discriminating layer bear R groups which are exclusively Br, Cl, or mixtures thereof, said gas separation membrane exhibiting a separation factor for oxygen and nitrogen at the ambient temperature of at least 6.1; and

(b) removing the permeated gas from the other side of the membrane.

For the purpose of the present invention the ambient temperature is a temperature of from 20 ° C to 24 ° C inclusive.

The membranes used in this invention demonstrate surprisingly high separation factors for oxygen and nitrogen separations. Further, the membranes used in this invention demonstrate surprisingly high separation factors for the separation of carbon dioxide from methane. The membranes used in this invention have good mechanical properties and therefore are useful under more extreme conditions, for example temperature and pressure.

The membranes used in this invention are prepared from polycarbonates derived from bisphenols wherein a significant portion of the bisphenols used to prepare the polycarbonates are tetrahalo-substituted, more particularly the tetrahalo-substituents are found in the 3,5-positions on the aromatic or phenolic rings. The presence of a significant portion of the residue of tetrahalo bisphenols enhance the membrane properties of membranes that are prepared therefrom. More particularly, such membranes have enhanced separation factors with respect to oxygen/nitrogen separations and carbon dioxide/methane separations.

Preferably, at least 30 weight percent of the moieties derived from the bisphenol of formula I present in the discriminating layer bear R groups which are exclusively bromine, chlorine, or mixtures thereof. More preferably, at least 50 weight percent of the moieties derived from the bisphenol of formula I present in the discriminating layer bear R groups which are exclusively bromine, chlorine, or mixtures thereof. Even more preferably, at least 70 weight percent of the moieties derived from the bisphenol of formula I present in the discriminating layer bear R groups which are exclusively bromine, chlorine, or mixtures thereof. Even more preferably, the polycarbonate is derived from bisphenols of formula I, where R is exclusively bromine, chlorine, or mixtures thereof. In the embodiment wherein the polycarbonate is prepared from tetrachlorobisphenols, it is preferable that the polycarbonate backbone contain 90 percent by weight or greater units derived from tetrachlorobisphenols, more preferably 95 percent by weight, and most preferably 100 percent by weight. Bromine is the preferred halogen herein. Examples of preferred bisphenols of formula I which bear R groups which are exclusively Br or Cl are 2,2-bis(3,5-dibromo-4-hydroxyphenyl)propane and 2,2-bis(3,5-dichloro-4-hydroxyphenyl)propane with 2,2-bis(3,5-dibromo-4-hydroxyphenyl)propane being most preferred.

The polycarbonates used in this invention preferably have recurring units which correspond to the formula

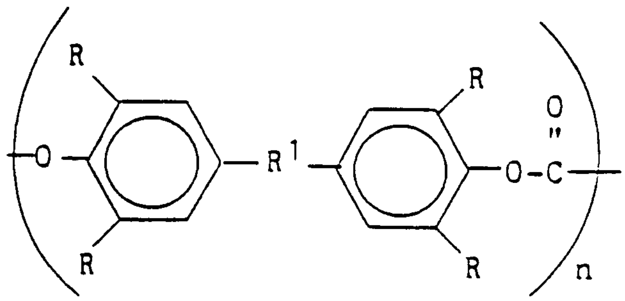

(II)

wherein R and $R^1$ are as hereinbefore defined and n is an integer of 50 or greater.

Examples of bisphenols within the scope of formula I wherein R groups are not Br or Cl, include 2,2-bis-(4-hydroxyphenyl)propane, 2,2-bis(3,5-dimethyl-4-hydroxyphenyl)propane, and the like. Methyl is the most preferred $C_{1-4}$ alkyl in bisphenols of formula I wherein R groups are $C_{1-4}$ alkyl.

In the hereinbefore presented formulae, R is preferably chlorine, bromine or $C_{1-4}$ alkyl, more preferably chlorine, bromine or methyl, even more preferably chlorine and bromine, and most preferably bromine. $R^1$ is preferably a $C_{1-6}$ divalent hydrocarbon, more preferably a $C_{1-6}$ alkylidene radical, even more preferably a propylidene radical. The polycarbonates used in this invention can be prepared by any process known in the art which prepares polycarbonates with suitable properties for membrane formation. See Encyclopedia of Polymer Science & Technology, Editor Mark et al, Interscience Division of John Wiley & Sons, N.Y., N.Y., 1969, Vol. 10, pp. 714-725. The polymers used in this invention should be polymerized to the extent that the polymers will form a membrane with sufficient mechanical strength to withstand use conditions. Preferably, the polymer has an inherent viscosity of 0.40 dL/g (0.040 $m^3$/kg), or greater, and preferably has a molecular weight of 60,000 or greater.

The membranes used in this invention can take any form known to one skilled in the art. In particular, the membrane may be a homogeneous membrane, a composite membrane, or an asymmetric membrane. Furthermore, the membranes may be in the form of a flat sheet, a hollow tube, or a hollow fiber. One skilled in the art would readily know how to prepare a membrane in any of the aforementioned forms. As used herein, the term semi-permeable membrane refers to a membrane which displays different permeabilities for different species of molecules, and therefore, may be used in the separation of ions and molecules having different permeabilities across the membrane. Permeate as used herein refers to those species which permeate through the membrane at a much faster rate than other species. Non-permeate refers herein to those species which permeate at a much slower rate than the other species present.

Preferably, the membranes used in this invention are asymmetric or composite membranes, and most preferably asymmetric membranes.

Homogeneous and composite membranes are prepared by forming a thin, discriminating layer which is dense and free of voids and pores. Such membranes or layers have generally the same structure and composition throughout the membrane. In one preferred embodiment, the polycarbonates used in this invention are dissolved in a water-miscible solvent, for example dimethylformamide, N-methyl pyrrolidinone, or dimethylacetamide. Preferably, the solution contains polymer in weight percents of between 5 and 75, more preferably between 10 and 40, and most preferably between 15 and 20 percent. This solution should have sufficient viscosity to allow casting of the solution onto a flat surface. The solution should be homogeneous. Thereafter, the polymer is cast on a surface, and in the case of a homogeneous membrane on a surface from which the finished membrane may readily be separated. A convenient way of carrying out this operation is either by casting the membrane solution onto a support surface which may be dissolved away from the finished film following the drying and curing step or by casting the membrane onto a support having low surface energy, such as silicone, coated glass, or a surface to which the membrane will not adhere, such as mercury. Casting is done by pouring the solution onto the appropriate surface and sizing using the appropriate tool, to form a solution of the appropriate thickness. Thereafter, the cast solution is exposed to drying or curing conditions. Such conditions are used to remove the solvent thereby leaving a thin, discriminating layer of polymer which is homogeneous. The solution can be dried either by exposure to a vacuum, exposure to elevated temperatures, by allowing the solvent to evaporate by time, or any combination thereof. Generally, it is preferable to expose the cast solution to elevated temperatures, preferably less than 100°C. In one preferred embodiment, such exposure is done in a vacuum oven or under vacuum conditions at elevated temperatures. Preferably, the homogeneous membrane has a

thickness of between 0.5 and 10.0 mils (0.01 and 0.25 mm), and most preferably between 1 and 3 mils (0.025 and 0.08 mm).

To prepare a composite membrane, a homogeneous thin, discriminating layer can be formed, and thereafter adhered to a porous support after formation. Alternatively, the porous support can be the surface upon which the membrane is cast. In such embodiment, composite membrane is prepared by casting a forming solution as a uniform coating on the porous support which forms the support layer for the finished membrane. Penetration of the polymer from which the thin, discriminating layer is formed into pores of the porous supporting layer and the layer itself is acceptable so long as the desired thickness of the semi-permeable membrane is not exceeded. In a composite membrane, the membrane is supported on a porous substrate or structure. This porous supporting layer is characterized in that it does not greatly impede the transport across this layer of all components of a fluid in contact with the porous layer. The porous supporting layer can comprise a discriminating layer which impedes the transportation of some fluid components to the discriminating layer, but generally this second discriminating layer is not necessary or desirable. In one embodiment, the supporting layer can be a metal or polymeric plate with a plurality of holes drilled through it. However, such a drill plate is not advantageous because it can significantly reduce the effective area of the membrane. In a preferred embodiment, the porous supporting layer is a very porous polymer membrane. Illustrative of such polymeric supporting layers are cellulose ester and microporous polysulfone membranes. Such membranes are commercially available under the trade names MILLIPORE, PELLICON and DIAFLOW . Where such supporting membranes are thin or highly deformable, a frame may also be necessary to adequately support the semi-permeable membrane. In one especially preferred embodiment, the polymeric supporting layer is a hollow fiber of a microporous polymer such as polysulfone, cellulose acetate, or some other cellulose ester. The hollow fiber itself provides adequate support for the semi-permeable membrane layer coated on the inside or outside surface of the fiber. Polysulfone hollow fibers are most preferred for this application. After the solution useful in forming the thin, discriminating layer is cast on the porous support, the porous support and solution cast thereon are then exposed to conditions for removal of the solvent so as to form the dense skin. Such conditions are similar to those described hereinbefore for the formation of the homogeneous membrane.

To form an asymmetric membrane, a solution is cast as described hereinbefore, and thereafter the cast solution is partially cured to remove a portion of the solvent. Thereafter, one or both surfaces of the partially dried membrane is contacted with a water quench so as to form a thin, non-porous, discriminating layer on one or both sides of the membrane under conditions such that the solvent away from the dense layer communicates to the dense layer forming pores in the remainder of the membrane, thereby forming an asymmetric membrane. Such porous layer is present to provide support for the thin, discriminating layer without impeding the transport of the fluid containing the components to be separated through the semi-permeable, thin, discriminating layer. The partial curing step is performed in a manner similar to the curing step described with respect to the formation of homogeneous membranes.

Hollow fiber membranes can be formed by spinning fibers from an appropriate solution of the polycarbonate in a water-miscible solvent. Such spinning is well known to those skilled in the art, and the formation of hollow fibers which are homogeneous, asymmetric, or composite membranes, requires the adaptation of the hereinbefore described procedures to the hollow fiber form of the membrane. Such adaptations are well within the skill of the art.

Generally, the thin, discriminating layer in a composite or asymmetric form of a membrane has a thickness of between 0.05 $\mu$m and 10 $\mu$m, more preferably between 0.2 $\mu$m and 2$\mu$m.

In one preferred embodiment, the membranes are annealed before use. It is believed that annealing increases the separation factor for oxygen-nitrogen separations. The membrane is exposed to temperatures above the beta transition and below the glass transition temperature for a period of time to partially densify the polymer. This procedure can optionally be performed under vacuum. For tetrabromo bisphenol A, temperatures between 185 °C and 230 °C are preferred.

Under certain conditions, it may be highly desirable to provide support to the membrane when the membrane is employed in a separation apparatus or process. In one embodiment, the peripheral area of the membrane is affixed to a framing structure which supports the outer edge of the membrane. The membrane can be affixed to the framing structure by a clamping mechanism, adhesive, chemical bonding, or other techniques known in the prior art. The membrane affixed to the frame can then be sealingly engaged in the conventional manner in a vessel so that the membrane surface inside the framing support separates two otherwise non-communicating compartments in the vessel. The skilled artisan will recognize that the structure which supports the membrane can be an integral part of the vessel or even the outer edge of the membrane.

In one embodiment, this invention is a process for separating oxygen from nitrogen which comprises

contacting a gaseous stream containing oxygen and nitrogen with the membrane of this invention under conditions such that oxygen selectively permeates through the membrane, in comparison to nitrogen. Preferably, the membrane is sealingly engaged to a vessel which defines a space communicating with only one side of the membrane, such that the permeable oxygen contacting the other side of the membrane can permeate through the membrane to the non-communicating space, at a significantly faster rate than the nitrogen communicates or permeates through the membrane. Preferably, the oxygen and nitrogen are a part of an air stream. Preferably, the pressure on the communicating side of the membrane is between 40 psia (275 kPa) and 200 psia (1375 kPa), more preferably between 80 (550 kPa) and 120 psia (825 kPa). The temperature at which the mixed oxygen and nitrogen stream is contacted with the membrane is between 0 and 80 ° C, most preferably between 5 and 45 ° C. The pressure differential across the membrane is preferably between 40 psia (275 kPa) and 200 psia (1375 kPa), and more preferably between 95 psia (650 kPa) and 120 psia (825 kPa). In one preferred embodiment, the membrane is in a hollow fiber form. In the embodiment wherein the membrane is in hollow fiber form, it is preferable to contact the mixed nitrogen and oxygen stream with a membrane on the outside of the hollow fiber under conditions such that the oxygen selectively permeates into the hollow fibers and a stream which is rich in oxygen is taken off of the end of the hollow fiber. This oxygen enriched stream can be further oxygen enriched by contacting with one or more membranes in succession.

In a similar manner, the membranes used in this invention can be used to separate carbon dioxide from methane.

In that embodiment wherein at least 35 weight percent of the moieties derived from formula I present in the discriminating layer bear R groups which are exclusively bromine, the gas separation membrane exhibits a separation factor for oxygen over nitrogen of an ambient temperature of at least 6.4. In that embodiment wherein the discriminating layer is derived exclusively from bisphenols in which the R is exclusively Br, the separation factor is at least 7.0 at 20 ° C. In that embodiment wherein the discriminating layer is derived exclusively from bis-phenols in which R is exclusively chlorine, the separation factor is at least 6.4 at 20 ° C.

In certain embodiments, the separations of oxygen from nitrogen occurs at lower temperatures, preferably at 10 ° C or below. It has been discovered that the membranes useful in this invention have surprisingly high separation factors at 10 ° C or below. Such separation factors are preferably 8.0 or greater, more preferably 8.5 or greater, and even more preferably 9.0 or greater at 10 ° C or below.

The following examples are included for illustrative purposes only and do not limit the scope of the claims or the invention. Unless otherwise stated, all parts and percentages are by weight.

Example 1 - Membrane of 100% Tetrabromobisphenol A Polycarbonate

Polymerization Procedure

A three neck, 1.0 liter round-bottom flask, equipped with a thermometer, air-driven stirrer and glass funnel, was charged with 500 $cm^3$ of methylene chloride, 108.8 grams (0.2 moles) of 3,3',5,5'-tetrabromo bisphenol A, 0.3 grams (0.002 moles) of p-tertiary butyl phenol and 42 $cm^3$ (0.52 moles) of pyridine. The resultant clear, pale yellow solution was stirred under a nitrogen atmosphere for ten minutes. Moderate stirring was continued and 23.5 grams (0.238 moles) of phosgene were bubbled into the reaction over a forty-one minute period.

The pale yellow, turbid solution was then scavenged with methanol, neutralized with dilute hydrochloric acid and washed a second time with dilute hydrochloric acid. The slightly opaque solution was clarified by passing it through a DOWEX® MSC ion exchange resin bed and precipitated in methanol. The precipitated polymer was dried under vacuum at 80 ° C for 24 hours. The resultant polymer was found to have an inherent viscosity of 0.428 dL/g (0.0428 $m^3$/kg) at 25 ° C in methylene chloride and a Tg of 261 ° C.

Film Preparation and Testing Procedure

Two grams of polymer were dissolved in 18 grams of methylene chloride, passed through a course, fritted glass filter onto a clean glass plate and drawn down with a casting blade. The sample was covered until dry, removed from the glass plate and annealed under vacuum at 80 ° C for 48 hours.

From a cast film, a small disc was removed, the mean thickness and standard deviation were determined and the film was then placed in the cell of a fixed volume-variable pressure gas permeability apparatus. Both sides of the membrane were evacuated overnight. One side of the membrane was pressurized with nitrogen at 184 kPaG and the downstream pressure increase was monitored with a

pressure transducer and recorded on a single-pen recorder. Gas permeability coefficients were calculated from the slope of the time-pressure curve.

Identical procedures were followed with each gas tested using the following sequence of test gases: nitrogen, methane, nitrogen, oxygen, helium, carbon dioxide. The results are compiled in the Table.

Example 2 - Polycarbonate Membrane of 100% Tetrachlorobisphenol A

In the apparatus similar to the one described in Example 1 (2.0 liters) was charged 1,000 $cm^3$ of methylene chloride: 183.2 grams (0.5 moles) of 3,3',5,5'-tetrachlorobisphenol A, and 105 $cm^3$ (1.30 moles) of pyridine. The resultant solution was stirred under a nitrogen atmosphere for 10 minutes. Moderate stirring was continued and 54 grams (0.55 moles) of phosgene was bubbled into the reaction over a 41 minute period.

The solution was then scavenged with methanol, neutralized with dilute hydrochloric acid and washed a second time with dilute hydrochloric acid. The slightly opaque solution was clarified by passing it through a Dowex® MSC ion exchange resin bed and precipitated in heptane. The precipitated polymer was dried under vacuum at 80°C for 24 hours. The resultant polymer was found to have an inherent viscosity of 0.72 dL/g (0.072 $m^3$/kg) at 25°C in methylene chloride.

A film was prepared and its permeation characteristics are tested as described in Example 1. The results were compiled in the Table.

Example 3 - Polycarbonate Membrane of 30% Tetrachlorobisphenol A and 70% Bisphenol A

A film was prepared from a polycarbonate with an inherent viscosity of 0.67, which is a block copolymer derived from 30 percent tetrachlorobisphenol A and 70 percent bisphenol A, and its permeation characteristics were tested as described in Example 1. The results were compiled in the Table.

Example 4 - Polycarbonate Membrane of 30% Tetrabromobisphenol A and 70% Bisphenol A

A four-neck, 2.0 liter round bottom flask equipped with a thermometer, air-driven stirrer, and glass funnel was charged with 70.14 grams (0.129 moles) of tetrabromobisphenol A and 1.181 liters of methylene chloride. The mixture was cooled to 5°C and stirred at 250 rpm's. 25.5 g (0.258 moles) of phosgene was added over eight minutes at 5 to 6°C. After five minutes, 20.9 milliliters of pyridine was added over a six minute period, with stirring at 300 rpm's at 5 to 6°C. The solution was stirred for 30 minutes. Bisphenol A 68.69 g (0.301 moles) was added to the solution. Thereafter, 69.5 milliliters pyridine was added over a five minute period and the temperature went from 13 to 24°C. Phosgene (17.0 g) was added over a 15 minute period at a temperature of 26 to 27°C. Thereafter, five milliliters of methanol was added. A mixture of HCl and water (490 milliliters and 120 milliliters respectively) was added over five minutes with stirring at 200 rpm's. Good phase separation was observed. A second wash was performed with a mixture of 15 milliliters HCl and 230 milliliters water.

The polymer was precipitated by adding one volume of polymer solution to four volumes of hexane. The polymer was air dried, then dried in a vacuum oven at 120°C. The resultant polymer has an inherent viscosity of 0.474 dl/g (0.0474 $m^3$/kg) and a Tg of 181°C.

A film was prepared and its permeation characteristics were tested as described in Example 1. The results are compiled in the Table.

Example 5 - Not An Example of This Invention - Membrane of 100% Bisphenol A

In the apparatus similar to that described in Example 1 (12.0 liters) was charged 7,000 $cm^3$ of methylene chloride, 961 grams (4.2 moles) of bisphenol A, 12.66 grams of p-tertiary butyl phenol and 882 $cm^3$ (10.9 moles) of pyridine. The resultant solution was stirred under a nitrogen atmosphere for 10 minutes. Moderate stirring was continued and 446 grams (4.51 moles) of phosgene was bubbled into the reaction over a 300 minute period.

The solution was then scavenged with methanol, neutralized with dilute hydrochloric acid and washed a second time with dilute hydrochloric acid. The slightly opaque solution was clarified by passing it through a Dowex® MSC ion exchange resin bed and precipitated in methanol. The precipitated polymer was dried under vacuum at 80°C for 24 hours. The resultant polymer was found to have an inherent viscosity of 0.61 dL/g (0.061 $m^3$/kg) at 25°C in methylene chloride.

A film was prepared and its permeation characteristics were tested as described in Example 1. The

results were compiled in the Table.

Example 6 - Polycarbonate Membrane of 100% 3,3',5,5' Tetramethyl Bisphenol A - Not An Embodiment of This Invention

In the apparatus as described in Example 1 was charged 500 $cm^3$ of methylene chloride, 113.7 grams (0.4 moles) of 3,3',5,5'-tetramethyl bisphenol A, and 110 $cm^3$ (1.36 moles) pyridine. The resultant solution was stirred under a nitrogen atmosphere for 10 minutes. Moderate stirring was continued and 46 grams of (0.46 moles) of phosgene was bubbled into the reaction over a 140 minute period.

The solution was then scavenged with methanol, neutralized with dilute hydrochloric acid and washed a second time with dilute hydrochloric acid. The slightly opaque solution was clarified by passing it through a Dowex® MSC ion exchange resin bed and precipitated in methanol. The precipitated polymer was dried under vacuum at 80 ° C for 24 hours. The resultant polymer was found to have an inherent viscosity of 0.456 dL/g (0.0456 $m^3$/kg) at 25 ° C in methylene chloride.

A film was prepared and its permeation characteristics were tested as described in Example 1. The results are compiled in the Table.

Example 7 - Polycarbonate Membrane of 50% 3,3'5,5'-Tetramethyl Bisphenol A and 50% Bisphenol A - Not An

Example of the Invention

A film of a polycarbonate derived from 50 mole percent tetramethyl bisphenol A and 50 mole percent bisphenol A with an inherent viscosity of 0.54 dL/g (0.054 $m^3$/kg) 25 ° C in methylene chloride was prepared and its permeation characteristics were tested as described in Example 1. The results are compiled in the Table.

Example 8 - Polycarbonate Membrane of 30% 3,3'5 5' Tetramethyl Bisphenol A and 70% Bisphenol A - Not An Example of This Invention

A film was prepared from a polycarbonate derived from 30 mole percent tetramethyl bisphenol A and 70 mole percent bisphenol A, with an inherent viscosity of 0.81 dL/g (0.081 $m^3$/kg) at 25 ° C in methylene chloride and its permeation characteristics were tested as described in Example 1. The results are compiled in the Table.

Example 9 - Polycarbonate Membrane of 70% 3,3',5,5'Tetrachlorobisphenol A and 30% Bisphenol A

In an apparatus similar to the one described in Example 1 (2.0 liters) was charged 1,000 $cm^3$ of methylene chloride, 117.7 grams (0.35 moles) of 3,3'5,5'-tetrachlorobisphenol A, 34.2 grams (0.15 moles) of bisphenol A, and 105 $cm^3$ (1.30 moles) of pyridine. The resultant solution was stirred under a nitrogen atmosphere for 10 minutes. Moderate stirring was continued and 54 grams (0.55 moles) of phosgene was bubbled into the reaction over a 70 minute period.

The solution was then scavenged with methanol, neutralized with dilute hydrochloric acid and washed a second time with dilute hydrochloric acid. The slightly opaque solution was clarified by passing it through a Dowex® MSC ion exchange resin bed and precipitated in methanol. The precipitated polymer was dried under vacuum at 80 ° C for 24 hours.

A film was prepared and its permeation characteristics were tested as described in Example 1. The results are compiled in the Table.

Example 10 - Polycarbonate Membrane of 70% 3,3'5,5'-Tetrabromo Bisphenol A and 30% Bisphenol A

In the apparatus similar to the one described in Example 1 (2.0 liters) was charged 1,000 $cm^3$ of methylene chloride, 190.4 grams (0.35 moles) of 3,3',5,5'-tetrabromobisphenol A, 34.2 grams (0.15 moles) of bisphenol A, and 105 $cm^3$ (1.30 moles) of pyridine. The resultant solution was stirred under a nitrogen atmosphere for 10 minutes. Moderate stirring was continued and 64 grams (0.65 moles) of phosgene was bubbled into the reaction over a 60 minute period.

The solution was then scavenged with methanol, neutralized with dilute hydrochloric acid and washed a second time with dilute hydrochloric acid. The slightly opaque solution was clarified by passing it through a

Dowex® MSC ion exchange resin bed and precipitated in methanol. The precipitated polymer was dried under vacuum at 80 °C for 24 hours. The resultant polymer was found to have an inherent viscosity of 0.53 dL/g (0.053 $m^3$/kg) at 25 °C in methylene chloride.

A film was prepared and its permeation characteristics were tested as described in Example 1. The results are compiled in the Table.

Example 11 - Polycarbonate Membrane of 15% 3,3'5,5'-Tetrabromo Bisphenol A and 85% Bisphenol A - Not An

Example of This Invention

In an apparatus similar to the one described in Example 1 was charged 1,000 $cm^3$ of methylene chloride, 40.8 grams (0.075 moles) of 3,3',5,5'-tetrabromobisphenol A, 97.0 grams (0.425 mole) of bisphenol A, and 105 $cm^3$ (1.30 mole) of pyridine. The resultant solution was stirred under a nitrogen atmosphere for 10 minutes. Moderate stirring was continued and 53 grams (0.54 mole) of phosgene was bubbled into the reaction over a 39 minute period.

The solution was then scavenged with methanol, neutralized with dilute hydrochloric acid and washed a second time with dilute hydrochloric acid. The slightly opaque solution was clarified by passing it through a Dowex® MSC ion exchange resin bed and precipitated in methanol. The precipitated polymer was dried under vacuum at 80 °C for 24 hours. The resultant polymer was found to have an inherent viscosity of 1.15 dL/g (0.115 $m^3$/kg) at 25 °C in methylene chloride.

A film was prepared and its permeation characteristics were tested as described in Example 1. The results are compiled in the Table.

Example 12 - Polycarbonate Membrane of 15% 3,3'5,5' Tetrachlorobisphenol A and 85% Bisphenol A - Not An

Example of This Invention

In an apparatus similar to the one described in Example 1 (2.0 liters) was charged 1,000 $cm^3$ of methylene chloride, 25.2 grams (0.75 mole) of 3,3',5,5'-tetrachlorobisphenol A, 9.70 grams (0.425 mole) of bisphenol A, and 105 $cm^3$ (1.30 mole) of pyridine. The resultant solution was stirred under a nitrogen atmosphere for 10 minutes. Moderate stirring was continued and 55 grams (0.56 mole) of phosgene was bubbled into the reaction over a 44 minute period.

The solution was then scavenged with methanol, neutralized with dilute hydrochloric acid and washed a second time with dilute hydrochloric acid. The slightly opaque solution was clarified by passing it through an MSC ion exchange resin bed and precipitated in methanol. The precipitated polymer was dried under vacuum at 80 °C for 24 hours. The resultant polymer was found to have an inherent viscosity of 0.819 dL/g (0.0819 $m^3$/kg) at 25 °C in methylene chloride.

A film was prepared and its permeation characteristics were tested as described in Example 1. The results are compiled in the Table.

Example 13 - Polycarbonate Membrane of 50% 3,3'5,5' Tetrabromobisphenol A and 50% Bisphenol A

In the apparatus similar as described in Example 1 (2.0 liters) was charged 500 $cm^3$ of methylene chloride, 54.4 grams (0.10 mole) of 3,3',5,5'-tetrabromobisphenol A, 22.8 grams (0.10 mole) of bisphenol A, and 42 $cm^3$ (0.52 mole) of pyridine. The resultant solution was stirred under a nitrogen atmosphere for 10 minutes. Moderate stirring was continued and 28 grams (0.28 mole) of phosgene was bubbled into the reaction over a 36 minute period.

The solution was then scavenged with methanol, neutralized with dilute hydrochloric acid and washed a second time with dilute hydrochloric acid. The slightly opaque solution was clarified by passing it through an MSC ion exchange resin bed and precipitated in methanol. The precipitated polymer was dried under vacuum at 80 °C for 24 hours. The resultant polymer was found to have an inherent viscosity of 0.39 dL/g (0.039 $m^3$/kg) at in methylene chloride and a Tg of 203 °C.

A film was prepared and its permeation characteristics were tested as described in Example 1. The results were compiled in the Table.

Example 14 - Polycarbonate Membrane of 50% 3,3'5,5' Tetrachlorobisphenol A and 50% Bisphenol A

In the apparatus as described in Example 1 was charged 500 $cm^3$ of methylene chloride, 36.16 grams (0.10 mole) of 3,3',5,5'-tetrachlorobisphenol A, 22.8 grams (0.10 mole) of bisphenol A, and 42.0 $cm^3$ (0.52 mole) of pyridine. The resultant solution was stirred under a nitrogen atmosphere for 10 minutes. Moderate stirring was continued and 28 grams (0.28 mole) of phosgene was bubbled into the reaction over a 48 minute period.

The solution was then scavenged with methanol, neutralized with dilute hydrochloric acid and washed a second time with dilute hydrochloric acid. The slightly opaque solution was clarified by passing it through an MSC ion exchange resin bed and precipitated in methanol. The precipitated polymer was dried under vacuum at 80 ° C for 24 hours. The resultant polymer was found to have an inherent viscosity of 0.51 dL/g (0.051 $m^3$/kg) at 25 ° C in methylene chloride and a Tg of 177 ° C.

A film was prepared and its permeation characteristics were tested as described in Example 1 with the exception that the film was not annealed to completely remove the residual solvent. This resulted in a residual solvent interfering with the transfer properties of the membrane. The results are compiled in the Table.

Table 1

| Example | Bisphenol | %[1] | $P^4O$ | $P^4N$ | $\alpha^5 O_2/N_2$ | $P^4CO_2$ | $P^4CH_4$ | $\alpha^5 CO_2/CH_4$ |
|---|---|---|---|---|---|---|---|---|
| 1 | Tetrabromo Bisphenol A | 100 | 0.8 (0.6) | 0.108 (0.081) | 7.4 | 3.6 (2.7) | 0.103 (0.077) | 35.0 |
| 2 | Tetrachloro Bisphenol A | 100 | 1.448 (1.086) | 0.231 (0.173) | 6.3 | 2.6 (1.95) | 0.103 (0.077) | 25.2 |
| 3 | Tetrachloro Bisphenol A | 30 | 0.8 (0.6) | 0.131 (0.098) | 6.1 | 3.6 (2.7) | 0.113 (0.085) | 31.9 |
| 4 | Tetrabromo Bisphenol A | 30 | 0.8 (0.6) | 0.125 (0.094) | 6.4 | 3.6 (2.7) | 0.112 (0.084) | 32.1 |
| 5[2] | Bisphenol A | 0 | 1.1 (0.8) | 0.208 (0.156) | 5.3 | 5.5 (4.1) | 0.228 (0.171) | 24.1 |
| 6[2] | Tetramethyl Bisphenol A | 100 | 3.9 (2.9) | 0.78 (0.585) | 5.0 | 16.3 (12.2) | 0.610 (0.458) | 26.7 |
| 7[2] | Tetramethyl Bisphenol A | 50 | 1.4 (1.05) | 0.233 (0.175) | 6.0 | 5.7 (4.3) | 0.241 (0.181) | 23.7 |

Table 1 (continued)

| Example | Bisphenol | %[1] | $P^4O$ | $P^4N$ | $\alpha^5 O_2/N_2$ | $P^4CO_2$ | $P^4CH_4$ | $\alpha^5 CO_2/CH_4$ |
|---|---|---|---|---|---|---|---|---|
| 8[2] | Tetramethyl Bisphenol A | 30 | 1.3 (1.0) | 0.224 (0.168) | 5.8 | 5.7 (4.28) | 0.206 (0.155) | 27.7 (20.8) |
| 9 | Tetrachloro Bisphenol A | 70 | 1.34 (1.00) | 0.228 (0.171) | 5.9 | | | |
| 10 | Tetrabromo Bisphenol A | 70 | 0.93 (0.70) | 0.14 (0.11) | 6.6 | | | |
| 11[2] | Tetrabromo Bisphenol A | 15 | 1.09 (0.82) | 0.19 (0.14) | 5.7 | | | |
| 12[2] | Tetrachloro Bisphenol A | 15 | 1.02 (0.77) | 0.21 (0.16) | 4.9 | | | |
| 13 | Tetrabromo Bisphenol A | 50 | 0.98 (0.74) | 0.15 (0.11) | 6.5 | 4.61 (3.46) | 0.16 (0.12) | 28.8 (21.6) |
| 14 | Tetrachloro Bisphenol A | 50 | 1.24 (0.93) | 0.23 (0.17) | 5.4 | 5.49 (4.12) | 0.22 (0.17) | 25.0 (18.8) |

1. Remainder is Bisphenol A
2. Not an embodiment of the invention
3. Example 5 is 100% bisphenol A polycarbonate
4. P is permeability measured in barrers ($cm^3$-$cm^2$-s-cmHg) x $10^{10}$ (($cm^3$-$cm^2$-s-kPa) x $10^{10}$)
5. $\alpha$ is a separation factor at ambient temperature

Example 15 -Polycarbonate Membrane of 50% 3,3'-,5,5'Tetra-methyl Bisphenol A and 50% 3,3',5,5'-Tetrabromobisphenol A

A three-neck, 1 liter flask equipped with a thermometer, stirrer, and funnel was charged with 54.4 g (0.1

mole) of 3,3'5,5'-tetrabromobisphenol A, 28.4 g (0.1 mole) of 3,3'5,5'-tetramethyl bisphenol A, 50 $cm^3$ of pyridine, and 400 $cm^3$ of methylene chloride. Moderate stirring was initiated and 23 g of phosgene was bubbled into the reactor over a 5-1/2 hour period. The resultant pink turbid solution was then scavenged with methanol, neutralized with dilute HCl and washed a second time with dilute HCl. The slightly hazy solution was clarified by passing it through an MSC resin bed, and precipitated in methanol. The resultant polymer was found to have an inherent viscosity $\eta_{inh}$ = 0.48 dl/g (0.048 $m^3$/kg) in methylene chloride.

Two grams of the above sample were dissolved in 18 g of methylene chloride, filtered onto a glass plate and drawn down with a casting blade. The sample was covered until dry, removed from the glass plate and annealed at 80 ° C and 760 mm of Hg (100 kPa) for forty-eight hours.

From this clear sample, a small disc was removed and was found to have the following permeabilities and selectivities

| | |
|---|---|
| $PO_2$ | = 1.87 x $10^{-10}$ $cm^3$ /$cm^2$-sec-cm of Hg (1.40 x $10^{-10}$ $cm^3$/$cm^2$-sec-kPa) |
| $PN_2$ | = 0.27 x $10^{-10}$ $cm^3$ /$cm^2$-sec-cm of Hg (0.20 x $10^{-10}$ $cm^3$/$cm^2$-sec-kPa) |
| $\alpha O_2/N_2$ | = 6.9 |
| $PCH_4$ | = 0.23 x $10^{-10}$ $cm^3$ /$cm^2$-sec-cm of Hg (0.17 x $10^{-10}$ $cm^3$/$cm^2$-sec-kPa) |
| $PCO_2$ | = 7.01 x $10^{-10}$ $cm^3$ /$cm^2$-sec-cm of Hg (5.26 x $10^{-10}$ $cm^3$/$cm^2$-sec-kPa) |
| $\alpha CO_2/CH_4$ | = 30.5 |
| PHe | = 18.24 x $10^{-10}$ $cm^3$ /$cm^2$-sec-cm of Hg (13.68 x $10^{-10}$ $cm^3$/$cm^2$-sec-kPa) |
| $PCH_4$ | = 0.24 $cm^3$ /$cm^2$-sec-cm of Hg (0.18 x $10^{-10}$ $cm^3$/$cm^2$-sec-kPa |
| $\alpha He/CH_4$ | = 76. |

P is permeability

## Claims

**1.** A method of separating a gas from a gas mixture, comprising,

(a) contacting the gas mixture with one side of a gas separation membrane whilst maintaining a lower pressure on the other side of the membrane, said membrane comprising a thin discriminating layer of a carbonate polymer derived from a bisphenol corresponding to formula I

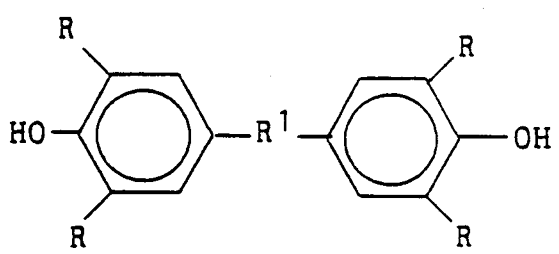

(I)

wherein R at each occurrence is independently H, Cl, Br, or $C_1$-$C_4$ alkyl and $R^1$ is -CO-,-S-,-$SO_2$-,-O-, or a $C_1$-$C_6$ divalent hydrocarbon radical, a $C_1$-$C_6$ divalent fluorocarbon radical or inertly substituted $C_1$-$C_6$ divalent hydrocarbon radical, with the proviso that at least 25 weight percent of the moieties derived from the bisphenol of formula I present in the discriminating layer bear R groups which are exclusively Br, Cl, or mixtures thereof said gas separation membrane exhibiting a separation factor for oxygen and nitrogen at the ambient temperature of at least 6.1; and

(b) removing the permeated gas from the other side of the membrane.

**2.** A method as claimed in Claim 1, wherein at least 30 weight percent of the moieties derived from the bisphenol of formula I present in the discriminating layer bear R groups which are exclusively Br, Cl, or mixtures thereof.

**3.** A method as claimed in Claim 2, wherein at least 50 weight percent of the moieties derived from the bisphenol of formula I present in the discriminating layer bear R groups which are exclusively Br, Cl, or mixtures thereof.

**4.** A method as claimed in Claim 3, wherein 100 weight percent of the moieties derived from the bisphenol of formula I present in the discriminating layer bear R groups which are exclusively Br, Cl, or

mixtures thereof.

5. A method as claimed in any one of Claims 1 to 5, wherein $R^1$ is a $C_{1-6}$ divalent hydrocarbon radical.

6. A method as claimed in Claim 4, wherein the moiety derived from the bisphenol of formula I is tetrabromobisphenol A and the gas separation membrane has a separation factor of at least 7.0 at the ambient temperature.

7. A method as claimed in any one of the preceding claims, wherein the membrane has been annealed by heating to a temperature above the beta transition temperature and below the glass transition temperature to partially densify the polymer.

8. A method as claimed in any one of the preceding claims, wherein the gas mixture contains oxygen and nitrogen and the permeated gas is oxygen.

9. A method as claimed in any one of Claims 1 to 7, wherein the gas mixture contains carbon dioxide and methane and the permeated gas is carbon dioxide.

10. A method as claimed in any one of the preceding claims, wherein the gas mixture is contacted with the membrane at the temperature of from 0° to 80°C and the pressure difference across the membrane is maintained between 275 kPa (40 psia) and 1375 kPa (200 psia).

11. A method as claimed in Claim 10, wherein the pressure difference across the membrane is maintained between 650 kPa (95 psia) and 825 kPa (120 psia).

12. A method as claimed in Claim 10 or Claim 11, wherein the gas mixture is contacted with the membrane at a temperature of from 5° to 45°C.

13. Use of a membrane as defined in any one of the preceding claims to separate gases by selective permeation therethrough.

**Patentansprüche**

1. Verfahren zum Abtrennen eines Gases aus einer Gasmischung durch
(a) Inberührungbringen der Gasmischung mit einer Seite einer Gastrennmembran, während ein niedriger Druck an der anderen Seite der Membran aufrechterhalten wird, wobei die Membran eine dünne Trennschicht aus einem Carbonatpolymer enthält, das sich von einem Bisphenol entsprechend der Formel I ableitet

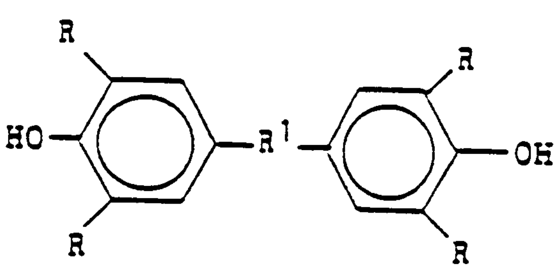

(I)

wobei R bei jedem Auftreten unabhängig voneinander H, Cl, Br oder $C_1$-$C_4$ Alkyl ist und $R^1$ -CO-, -S-, -$SO_2$-, -O- oder ein zweiwertiger $C_1$-$C_6$ Kohlenwasserstoffrest, ein zweiwertiger $C_1$-$C_6$ Fluorkohlenstoffrest oder ein mit inerten Substituenten versehener zweiwertiger $C_1$-$C_6$ Kohlenwasserstoffrest ist, unter der Voraussetzung, daß mindestens 25 Gew.-% der Einheiten, die sich von dem Bisphenol der Formel I ableiten und in der Trennschicht vorhanden sind, R-Gruppen tragen, die ausschließlich Br, Cl oder Mischungen davon sind, diese Gastrennmembran einen Trennfaktor für Sauerstoff und Stickstoff bei Umgebungstemperatur von wenigstens 6,1 hat, und
(b) Entfernen des hindurchgetretenen Gases von der anderen Seite der Membran.

2. Verfahren nach Anspruch 1, **dadurch gekennzeichnet**, daß von den in der Trennschicht vorhandenen Einheiten, die sich von dem Bisphenol nach Formel I ableiten, wenigstens 30 Gew.-% R-Reste tragen, die ausschließlich Br, Cl oder Mischungen davon sind.

3. Verfahren nach Anspruch 2, **dadurch gekennzeichnet**, daß von den in der Trennschicht vorhandenen Einheiten, die sich von dem Bisphenol nach Formel I ableiten, wenigstens 50 Gew.-% R-Reste tragen, die ausschließlich Br, Cl oder Mischungen davon sind.

4. Verfahren nach Anspruch 3, **dadurch gekennzeichnet**, daß von den in der Trennschicht vorhandenen Einheiten, die sich von dem Bisphenol nach Formel I ableiten, wenigstens 100 Gew.-% R-Reste tragen, die ausschließlich Br, Cl oder Mischungen davon sind.

5. Verfahren nach einem der Ansprüche 1-5, **dadurch gekennzeichnet**, daß $R^1$ ein zweiwertiger $C_{1-6}$ Kohlenwasserstoffrest ist.

6. Verfahren nach Anspruch 4, **dadurch gekennzeichnet**, daß die Einheit, die sich von dem Bisphenol nach Formel I ableitet, Tetrabromobisphenol A ist und die Gastrennmembran bei Umgebungstemperatur einen Trennfaktor von wenigstens 7,0 aufweist.

7. Verfahren nach einem der vorstehenden Ansprüche, **dadurch gekennzeichnet**, daß die Membran durch Erwärmen auf eine Temperatur oberhalb der beta Übergangstemperatur und unterhalb der Glasübergangstemperatur getempert worden ist, um das Polymer teilweise zu verdichten.

8. Verfahren nach einem der vorstehenden Ansprüche, **dadurch gekennzeichnet**, daß die Gasmischung Sauerstoff und Stickstoff enthält und das hindurchgetretene Gas Sauerstoff ist.

9. Verfahren nach einem der Ansprüche 1-7, **dadurch gekennzeichnet**, daß die Gasmischung Kohlendioxid und Methan enthält und das hindurchgetretene Gas Kohlendioxid ist.

10. Verfahren nach einem der vorstehenden Ansprüche, **dadurch gekennzeichnet**, daß die Gasmischung mit der Membran bei einer Temperatur von 0-80 °C in Berührung gebracht wird und die Druckdifferenz durch die Membran zwischen 275 kPa (40 psia) und 1375 kPa (200 psia) gehalten wird.

11. Verfahren nach Anspruch 10, **dadurch gekennzeichnet**, daß die Druckdifferenz durch die Membran zwischen 650 kPa (95 psia) und 825 kPa (120 psia) gehalten wird.

12. Verfahren nach Anspruch 10 oder 11, **dadurch gekennzeichnet**, daß die Gasmischung bei einer Temperatur von 5-45 °C mit der Membran in Berührung gebracht wird.

13. Verwendung der Membran, wie in einem der vorstehenden Ansprüche definiert, zur Trennung von Gasen durch selektive Durchlässsigkeit durch die Membran.

**Revendications**

**1.** Procédé de séparation d'un gaz d'avec un mélange de gaz, comportant :

(a) le fait de mettre le mélange de gaz en contact avec l'un des côtés d'une membrane de séparation de gaz, tout en maintenant une pression plus faible de l'autre côté de la membrane, ladite membrane comportant une couche mince sélective en un polycarbonate dérivé d'un bisphénol correspondant à la formule I

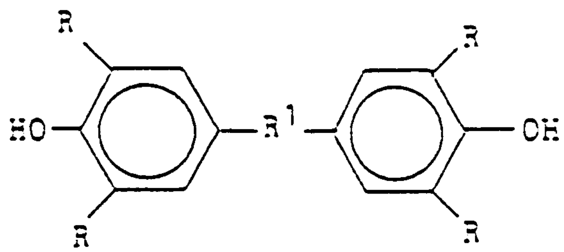

(I)

dans laquelle chaque R représente indépendamment H, Cl, Br, ou un groupe alkyle en $C_1$-$C_4$, et $R^1$ représente -CO-, -S-, -$SO_2$-, -O-, ou un radical hydrocarboné divalent en $C_1$-$C_6$, un radical fluorocarboné divalent en $C_1$-$C_6$ ou un radical hydrocarboné divalent en $C_1$-$C_6$ portant des substituants inertes, sous réserve qu'au moins 25 % en poids des motifs dérivés du bisphénol de formule I présents dans la couche sélective portent des groupes R qui sont exclusivement Br, Cl ou leurs mélanges, et ladite membrane de séparation de gaz présentant un facteur de séparation pour l'oxygène et l'azote, à la température ambiante, valant au moins 6,1 ; et

(b) l'enlèvement du gaz ayant traversé la membrane, de l'autre côté de celle-ci.

**2.** Procédé conforme à la revendication 1, dans lequel au moins 30 % en poids des motifs dérivés du bisphénol de formule I présents dans la couche sélective portent des groupes R qui sont exclusivement Br, CL ou leurs mélanges.

**3.** Procédé conforme à la revendication 2, dans lequel au moins 50 % en poids des motifs dérivés du bisphénol de formule I présents dans la couche sélective portent des groupes R qui sont exclusivement Br, Cl ou leurs mélanges.

**4.** Procédé conforme à la revendication 3, dans lequel 100 % en poids des motifs dérivés du bisphénol de formule I présents dans la couche sélective portent des groupes R qui sont exclusivement Br, Cl ou leurs mélanges.

**5.** Procédé conforme à l'une quelconque des revendications 1 à 5, dans lequel $R^1$ représente un radical hydrocarboné divalent en $C_1$-$C_6$.

**6.** Procédé conforme à la revendication 4, dans lequel le bisphénol de formule I est du tétrabromobisphénol A et la membrane de séparation de gaz présente un facteur de séparation valant au moins 7,0 à la température ambiante.

**7.** Procédé conforme à l'une quelconque des revendications précédentes, dans lequel on a fait subir à la membrane un recuit, en la chauffant à une température supérieure à la température de transition bêta et inférieure à la température de transition vitreuse, afin de densifier partiellement le polymère.

**8.** Procédé conforme à l'une quelconque des revendications précédentes, dans lequel le mélange gazeux contient de l'oxygène et de l'azote, et le gaz traversant la membrane est de l'oxygène.

**9.** Procédé conforme à l'une quelconque des revendications 1 à 7, dans lequel le mélange de gaz contient du dioxyde de carbone et du méthane, et le gaz traversant la membrane est du dioxyde de carbone.

**10.** Procédé conforme à l'une quelconque des revendications précédentes, dans lequel on met le mélange de gaz en contact avec la membrane à une température de 0° à 80°C, et la différence de pression

entre les deux côtés de la membrane est maintenue entre 275 kPa (40 psia) et 1375 kPa (200 psia).

**11.** Procédé conforme à la revendication 10, dans lequel la différence de pression entre les deux côtés de la membrane est maintenue entre 650 kPa (95 psia) et 825 kPa (120 psia).

**12.** Procédé conforme à la revendication 10 ou 11, dans lequel on met le mélange de gaz en contact avec la membrane à une température de 5° à 45°C.

**13.** Utilisation d'une membrane telle que définie dans l'une quelconque des revendications précédentes, pour séparer des gaz par perméation sélective à travers elle.